# FASCICULE DE BREVET EUROPEEN

(11) **EP 0 521 088 B1**
(45) Date de publication et mention de la délivrance du brevet: **09.03.1994**
(21) Numéro de dépôt: 91907070.6
(22) Date de dépôt: 20.03.1991
(51) Int. Cl.: A61B 1/32, A61B 1/00

(54) **INSTRUMENT POUR LA MISE EN OEUVRE D'INTERVENTIONS MEDICALES OU CHIRURGICALES PAR LAPAROSCOPIE OU COELIOSCOPIE**
INSTRUMENT ZUR DURCHFÜHRUNG VON MEDIZINISCHEN ODER CHIRURGISCHEN EINGRIFFEN PER LAPAROSKOPIE ODER COELIOSKOPIE
INSTRUMENT FOR PERFORMING MEDICAL OR SURGICAL OPERATIONS BY LAPAROSCOPY OR COELISCOPY

(30) Priorité: 20.03.1990 FR 9003980
(43) Date de publication de la demande: 07.01.1993
(73) Titulaire: MOURET, Philippe, F-01600 Trévoux (FR)
(72) Inventeur: MOURET, Philippe, F-01600 Trévoux (FR)
(74) Mandataire: Maureau, Philippe
(86) Numéro de dépôt international: FR9100227
(87) Numéro de publication internationale: WO9114392

(56) Documents cités:
- DE-C- 661 403
- FR-A- 2 303 512
- FR-A- 2 339 936

## Description

La présente invention a pour objet un instrument pour la mise en oeuvre d'interventions médicales ou chirurgicales par laparoscopie ou coelioscopie.

Il est fréquent, à des fins d'investigations médicales, de procéder par une technique de coelioscopie, qui consiste à réaliser la mise en pression du volume délimité par le péritoine, assurant un soulèvement de la paroi abdominale, avant introduction dans celle-ci d'un trocart permettant le passage d'un dispositif optique permettant de visualiser l'état des organes sur lesquels une investigation doit être effectuée.

Il est également connu d'utiliser cette technique pour réaliser des interventions chirurgicales. Dans un tel cas il convient de ménager des passages pour plusieurs trocarts dont certains servent à la visualisation des organes sur lesquels l'intervention doit être effectuée, et dont d'autres servent au passage des instruments.

Actuellement le soulèvement de la paroi est obtenu par un différentiel de pression suffisant entre la cavité péritonéale et l'extérieur. Toutefois ce différentiel de pression ne peut être augmenté sans danger en raison des perturbations circulatoires et ventilatoires que cela entraînerait.

Or la pluralité des voies de passage entraîne des fuites de gaz assez importantes pour interdire le maintien d'une pression suffisante à l'intérieur du péritoine. En effet, les appareils d'insufflation de pneumopéritoine possèdent un débit limité par construction, notamment pour des raisons de sécurité. En outre, I'élévation de pression à l'intérieur de la cavité péritonéale représente un danger certain dans le déroulement d'une laparoscopie.

Cette contrainte d'étanchéité relative nécessite que tous les instruments utilisés doivent subir une adaptation très spécifique de manière à obtenir une étanchéité aussi parfaite que possible dans le franchissement de la paroi. Cette contrainte technique extrêmement rigoureuse est un facteur très restrictif dans la création et la réalisation d'instruments chirurgicaux coelioscopiques.

Par ailleurs, l'apparition de techniques opératoires comportant l'ablation de volume tissulaire ou d'organes plus importants nécessite la création d'orifices d'extraction plus volumineux que ceux utilisés habituellement dans la laparoscopie classique. Ces orifices d'extraction sont générateurs de fuites considérables du pneumopéritoine qui entraînent un effondrement de la paroi, se traduisant par une disparition presque complète du volume de manoeuvre ainsi que de la zone de visibilité du praticien.

La présente invention vise à remédier à ces inconvénients en fournissant un instrument permettant d'assurer un maintien mécanique du soulèvement de la paroi du péritoine.

A cet effet, l'instrument qu'elle concerne est réalisé en un matériau filiforme et comprend une première partie s'étendant sensiblement dans un plan, destinée à être positionnée contre la face profonde de la paroi abdominale antérieure, et une seconde partie qui, destinée à traverser la paroi abdominale, est terminée par un dispositif d'accrochage.

Selon une première forme d'exécution, cet instrument est réalisé en un matériau filiforme et comprend une partie en forme générale d'anneau ouvert destiné à être positionné contre la face profonde de la paroi addominale antérieure, et un élément de suspension destiné à traverser la paroi abdominale et terminé par un dispositif d'accrochage.

En pratique, la cavité péritonéale est mise en pression, et équipée d'un trocart souple qui traverse la paroi abdominale. Par l'intermédiaire de ce trocart souple est introduit la partie en forme d'anneau de l'instrument, à partir de l'extrémité libre ouverte de celle-ci, jusqu'à ce que cette partie en forme d'anneau vienne se positionner contre la face profonde de la paroi abdominale antérieure. L'élément de suspension qui se termine par un dispositif d'accrochage, par exemple en forme de crochet, est alors fixé à un support tel qu'une potence solidaire de la table d'opération. Il est ainsi possible de réaliser un maintien mécanique de la paroi abdominale, qui ne dépend plus exclusivement de l'insufflation de gaz à l'intérieur de la cavité péritonéale.

Afin de faciliter l'introduction en forme d'anneau à travers la paroi abdominale, cette partie en forme d'anneau est disposée dans un plan légèrement gauchi, la zone d'extrémité de la branche formant la partie ouverte de l'anneau étant inclinée par rapport au plan de celui-ci, du côté opposé à l'élément de suspension.

La forme de l'anneau n'est pas nécessairement circulaire, mais adaptée au type de suspension qui doit être réalisé, la largeur de l'ouverture de l'anneau devant être suffisante pour permettre la traversée de la paroi sans traumatisme tissulaire.

Selon une autre caractéristique de l'invention l'instrument est réalisé en un matériau déformable élastiquement. Il peut s'agir d'acier inoxydable ou d'une matière synthétique. L'essentiel est que l'instrument soit réalisé dans un matériau suffisamment rigide pour remplir sa fonction, c'est-à-dire supporter une traction qui maintient le degré de soulèvement pariétal souhaité tout en étant suffisamment élastique et déformable pour faciliter les manoeuvres d'introduction dans la cavité et éviter les traumatismes tissulaires.

Selon une forme d'exécution, l'instrument est réalisé en un élément tubulaire susceptible d'être raccordé à une source d'alimentation en gaz de la cavité péritonéale. L'instrument joue alors une double fonction, d'une part de maintien mécanique de la paroi abdominale et d'autre part de conduit d'amenée de gaz assurant le maintien en surpression de cette cavité, pour éviter les entrées d'air.

Selon une autre caractéristique de l'invention, l'élément de suspension forme un angle aigu avec le plan de l'anneau. Cet angle permet de disposer la partie en forme d'anneau avec un angle par rapport à la paroi, afin de donner un maximum de champ de visibilité dans l'axe du regard du coelioscope.

Conformément à une autre caractéristique de l'invention, la partie en forme d'anneau située sensiblement dans un plan est raccordée à l'élément de suspension par un segment curviligne, dépassant du plan de l'anneau du côté opposé à celui duquel dépasse l'élément de suspension. Ce segment curviligne forme une partie en creux dans laquelle vient s'engager une partie de la paroi abdominale, permettant une stabilisation de l'instrument lorsque celui-ci est en traction, et évitant tout risque de glissement. Il est à noter de façon générale que toutes les courbes de l'instrument doivent être d'un rayon suffisant pour éviter les contraintes tissulaires lors des manoeuvres d'introduction.

Selon encore une autre caractéristique, I'élément de suspension est muni d'une poignée ou similaire permettant de manipuler facilement l'instrument au cours de la phase d'introduction de l'anneau à l'intérieur de la cavité péritonéale.

Conformément à une possibilité, l'instrument est réalisé en deux partie assemblable l'une à l'autre de façon amovible au niveau de l'élément de suspension. Cette caractéristique rend l'instrument démontable, ce qui est avantageux pour réaliser les opérations de nettoyage et également de stérilisation.

Selon une autre forme d'exécution, cet instrument comprend une branche tubulaire droite qui, destinée à traverser la paroi abdominale, est prolongée à l'une de ses extrémités par une branche formant un angle avec elle, et destinée à venir prendre appui contre la face profonde de la paroi abdominale et à son autre extrémité par un bras de suspension terminé par un dispositif d'accrochage, la branche tubulaire servant au logement et au guidage en rotation d'un arbre dont une extrémité est équipée d'au moins une branche déplaçable lors du pivotement de l'arbre entre une position dans laquelle elle est au contact de la branche et une position dans laquelle elle forme un angle avec celle-ci, et dont l'autre extrémité est équipée d'un levier de manoeuvre dépassant de la branche tubulaire.

De toute façon, l'invention sera bien comprise à l'aide de la description en référence au dessin schématique annexé représentant à titre d'exemples non limitatifs trois formes d'exécution de cet instrument :
Figure 1 est une vue en perspective d'un premier instrument ;
Figure 2 est une vue en perspective d'un second instrument ;
Figure 3 est une vue de l'instrument de figure 2 en position d'utilisation.
Figures 4 et 5 sont deux vues en perspective d'un autre instrument dans deux positions.

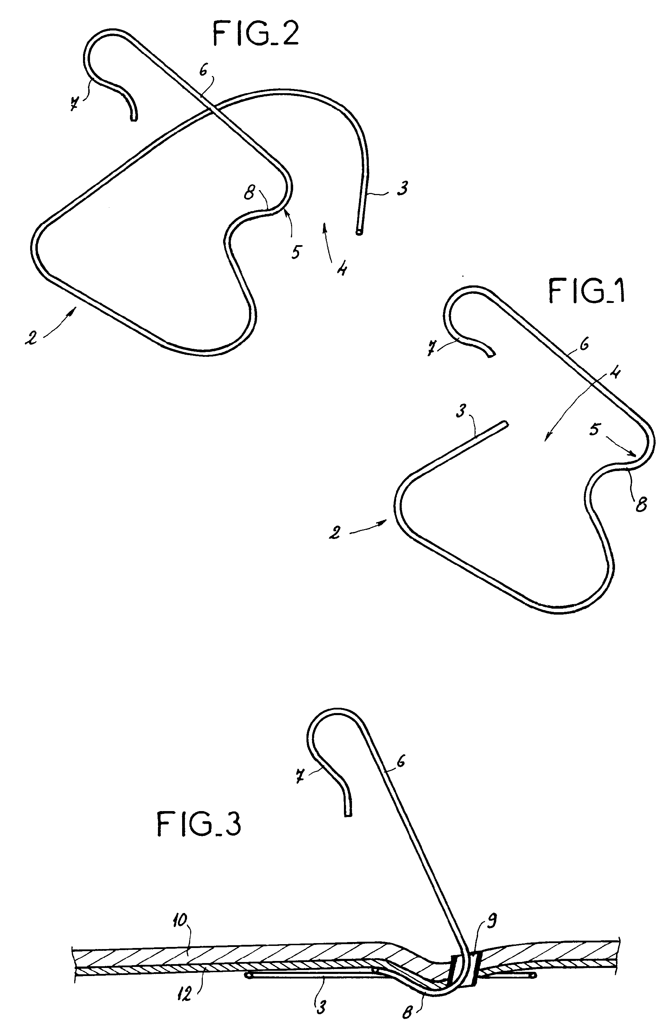

L'instrument représenté à la figure 1, comprend une partie 2 en forme d'anneau ouvert, située sensiblement dans un plan, dont l'extrémité libre appartient à une branche 3 légèrement inclinée par rapport au plan de l'anneau. L'extrémité libre délimite avec l'autre extrémité de l'anneau une ouverture 4 dont la largeur est au moins à l'épaisseur de la paroi abdominale. L'autre extrémité de l'anneau, désignée par la référence 5, est équipée d'un prolongement constitué par un élément 6 de suspension dont l'extrémité libre se termine par un crochet 7 destiné par exemple à être fixé sur une potence ou un autre support solidaire de la table d'opération. La partie de l'anneau située dans un plan est raccordée à l'élément de suspension par un segment curviligne 8 qui fait saillie par rapport au plan de l'anneau, du côté opposé à l'élément de suspension 6.

En pratique, l'instrument est engagé par sa branche 3 à l'intérieur d'un trocart souple 9 dans la cavité péritonéale, un mouvement étant exercé pour que la totalité de l'anneau vienne prendre appui contre la face antérieure de la paroi abdominale 10, comme montré à la figure 3. Au cours de cette opération, la cavité péritonéale, dont le péritoine 12 est représenté au dessin, est mise en surpression, après quoi cette surpression est coupée, la paroi abdominale demeurant en position suspendue sous la seule action de l'instrument selon l'invention.

Il ressort de la comparaison entre les figures 1 et 2, dans lesquelles les mêmes éléments sont désignés par les mêmes références, que l'anneau 2 peut posséder des formes différentes. Il est également intéressant de noter que l'élément de suspension 6 forme avec le plan de l'anneau ouvert 2 un angle aigu, de telle sorte que le soulèvement de la paroi peut être plus important dans la zone dans laquelle le coelioscope est orienté. Enfin, comme il ressort clairement de la figure 3, la paroi abdominale vient s'appuyer dans le fond du segment curviligne 8, ce qui évite tout risque de glissement de l'instrument contre cette paroi au cours de l'intervention.

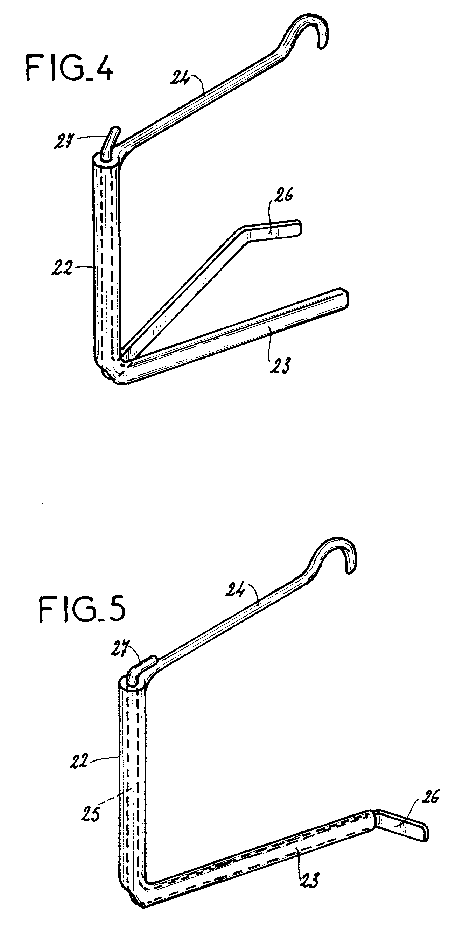

Les figures 4 et 5 représentent une autre forme d'exécution de cet instrument. Celui-ci comprend une branche tubulaire droite 22 qui, destinée à traverser la paroi abdominale, est prolongée à l'une de ses extrémités par une branche 23 formant un angle aigu avec elle, destinée à venir prendre appui contre la face profonde de la paroi abdominale. A son autre extrémité, la branche 22 est équipée d'un bras de suspension 24 terminé par un dispositif d'accrochage.

La branche tubulaire 22 sert au logement et au guidage en rotation d'un arbre 25 dont une extrémité est équipée d'une branche 26 disposée au niveau de la branche 23, et dont l'autre extrémité est équipée d'un levier de manoeuvre 27 dépassant de la branche tubulaire 22.

La branche 26 peut ainsi, par actionnement du levier 27, être pivotée entre une position, représentée à la figure 5, dans laquelle elle est jointive avec la branche 23, pour réaliser la traversée de la paroi addominale, et une position, représentée à la figure 4, dans laquelle elle forme un angle avec la branche 23, correspondant à la position de soutien de la paroi abdominale.

Il serait possible dans une variante d'envisager plus de deux branches pour le soutien de la paroi abdominale.

Dans les formes d'exécution représentées au dessin, l'instrument est constitué par un fil plein et réalisé en une seule pièce, par exemple en acier inoxyable. Toutefois cet instrument peut être équipé d'une poignée au niveau de l'élément de suspension, en vue de faciliter sa manutention, peut également être tubulaire pour permettre l'alimentation en gaz de la cavité péritonéale, ou peut également être réalisé en plusieurs parties démontables afin de faciliter ses conditions de stockage et de stérilisation.

Comme il ressort de ce qui précède, l'invention apporte une grande amélioration à la technique existante en fournissant un instrument assurant un soutien mécanique de la paroi abdominale, ce qui permet d'éviter d'avoir à maintenir pendant une période de temps importante une surpression à l'intérieur de la cavité péritonéale, cette surpression pouvant d'une part être difficile à maintenir dans la mesure où les fuites sont importantes, et pouvant causer au patient des perturbations circulatoires et ventilatoires.

Comme il va de soi l'invention ne se limite pas aux seules formes d'exécutions de cet instrument décrites ci-dessus à titre d'exemples, elle en embrasse, au contraire, toutes les variantes.

## Revendications

1. Instrument pour la mise en oeuvre d'interventions médicales ou chirurgicales par laparoscopie ou coelioscopie, caractérisé en ce qu'il présente une structure filiforme et comprend une première partie (2) s'étendant sensiblement dans un plan, destinée à être positionnée contre la face profonde de la paroi abdominale antérieure, et une seconde partie (6) qui, destinée à traverser la paroi abdominale, est terminée par un dispositif d'accrochage (7).

2. Instrument selon la revendication 1, caractérisé en ce qu'il est réalisé en un matériau filiforme et comprend une partie (2) en forme générale d'anneau ouvert destiné à être positionné contre la face profonde de la paroi abdominale antérieure, et un élément de suspension (6) destiné à traverser la paroi abdominale et terminé par un dispositif d'accrochage.

3. Instrument selon la revendication 2, caractérisé en ce que la partie (2) en forme d'anneau est disposée dans un plan légèrement gauchi, la zone d'extrémité (3) de la branche formant la partie ouverte de l'anneau étant inclinée par rapport au plan de celui-ci, du côté opposé à l'élément de suspension (6).

4. Instrument selon l'une quelconque des revendications 1 à 3, caractérisé en ce qu'il est réalisé en un matériau déformable élastiquement.

5. Instrument selon l'une quelconque des revendications 1 à 4, caractérisé en ce qu'il est réalisé en un élément tubulaire susceptible d'être raccordé à une source d'alimentation en gaz de la cavité péritonéale.

6. Instrument selon l'une quelconque des revendications 1 à 5, caractérisé en ce que l'élément de suspension (6) forme un angle aigu avec le plan de l'anneau (2).

7. Instrument selon l'une quelconque des revendications 2 à 6, caractérisé en ce que la partie (2) en forme d'anneau située sensiblement dans un plan est raccordée à l'élément de suspension (6) par un segment curviligne (8), dépassant du plan de l'anneau du côté opposé à celui duquel dépasse l'élément de suspension.

8. Instrument selon l'une quelconque des revendications 2 à 6, caractérisé en ce qu'il est muni d'une poignée ou similaire permettant de manipuler facilement l'instrument au cours de la phase d'introduction de l'anneau à l'intérieur de la cavité péritonéale.

9. Instrument selon l'une quelconque des revendications 2 à 8, caractérisé en ce qu'il est réalisé en deux parties assemblables l'une à l'autre de façon amovible au niveau de l'élément de suspension.

10. Instrument selon la revendication 1, caractérisé en ce qu'il comprend une branche tubulaire droite (22) qui, destinée à traverser la paroi abdominale, est prolongée à l'une de ses extrémités par une branche (23) formant un angle avec elle, et destinée à venir prendre appui contre la face profonde de la paroi abdominale et à son autre extrémité par un bras de suspension (24) terminé par un dispositif d'accrochage, la branche tubulaire (22) servant au logement et au guidage en rotation d'un arbre (25) dont une extrémité est équipée d'au moins une branche (26) déplaçable lors du pivotement de l'arbre (25) entre une position dans laquelle elle est au contact de la branche (23) et une position dans laquelle elle forme un angle avec celle-ci, et dont l'autre extrémité est équipée d'un levier de manoeuvre (27) dépassant de la branche tubulaire (22).

## Claims

1. An instrument for carrying out medical or surgical operations by laparoscopy or coelioscopy, characterised in that it has a wire-form structure and includes a first part (2) extending substantially in one plane, intended to be positioned against the deep face of the anterior abdominal wall, and a second part (6) which is intended to pass through the abdominal wall and terminates in a hook device (7).

2. An instrument according to Claim 1, characterised in that it is made from wire-form material and includes a part (2) in the general form of an open ring intended to be positioned against the deep face of the anterior abdominal wall, and a suspension element (6) intended to pass through the abdominal wall and terminating in a hook device.

3. An instrument according to Claim 2, characterised in that the part (2) in the form of a ring is disposed in a slightly skewed plane, the end region (3) of the branch forming the open part of the ring being inclined with respect to the plane of the latter, at the opposite side to the suspension element (6).

4. An instrument according to any one of Claims 1 to 3, characterised in that it is formed of a resiliently deformable material.

5. An instrument according to any one of Claims 1 to 4, characterised in that it is formed as a tubular element adapted to be connected to a source for supplying gas to the peritoneal cavity.

6. An instrument according to any one of Claims 1 to 5, characterised in that the suspension element (6) forms an acute angle with the plane of the ring (2).

7. An instrument according to any one of Claims 2 to 6, characterised in that the part (2) in the form of a ring situated substantially in one plane is connected to the suspension element (6) by a curved segment (8) extending out of the plane of the ring at the side opposed to that from which the suspension element extends.

8. An instrument according to any one of Claims 2 to 6, characterised in that it is provided with a handle or the like, enabling the instrument to be readily manipulated during the course of introduction of the ring into the interior of the peritoneal cavity.

9. An instrument according to any one of Claims 2 to 8, characterised in that it is made in two parts for assembly together in a removable manner at the level of the suspension element.

10. An instrument according to Claim 1, characterised in that it includes a straight tubular arm (22) which, intended to pass through the abdominal wall, is extended at one of its ends by an arm (23) forming an angle with it and intended to come to bear against the deep face of the abdominal wall and at its other end by a suspension arm (24) terminating in a hook device, the tubular arm (22) serving for the housing and guiding in rotation of a shaft (25) of which one end is provided with at least one arm (26) which is movable during pivoting of the arm (25) between a position in which it is in contact with the arm (23) and a position in which it forms an angle with the latter, and of which the other end is provided with a manipulating lever (27) extending beyond the tubular arm (22).

## Patentansprüche

1. Instrument zur Durchführung medizinischer oder chirurgischer Eingriffe durch Laparoskopie oder Coelioskopie, dadurch gekennzeichnet, daß es eine dünne Struktur aufweist und einen sich im wesentlichen in einer Ebene erstreckenden, zum Anliegen an der Innenseite der vorderen Bauchwand bestimmten ersten Abschnitt (2) umfaßt und einen zum Durchdringen der Bauchwand bestimmten zweiten Abschnitt (6), welcher in einem Eingriffsmittel (7) endet.

2. Instrument nach Anspruch 1, dadurch gekennzeichnet, daß es aus einem dünnen Material besteht und einen zum Anliegen an der Innenseite der vorderen Bauchwand bestimmten Abschnitt (2) mit der allgemeinen Form eines offenen Rings umfaßt und ein zum Durchdringen der Bauchwand bestimmtes Aufhängungselement (6), welches in einem Eingriffsmittel endet.

3. Instrument nach Anspruch 2, dadurch gekennzeichnet, daß der ringförmige Abschnitt (2) in einer leicht gekrümmten Ebene angeordnet ist, wobei der Endbereich (3) des den offenen Abschnitt des Rings bildenden Schenkels relativ zu dessen Ebene zu der dem Aufhängungselement (6) entgegengesetzten Seite hin geneigt ist.

4. Instrument nach einem der Ansprüche 1 bis 3, dadurch gekennzeichnet, daß es aus einem verformbaren, elastischen Material gebildet ist.

5. Instrument nach einem der Ansprüche 1 bis 4, dadurch gekennzeichnet, daß es aus einem rohrförmigen Element gebildet ist, welches dazu geeignet ist, mit einer Gaszufuhrquelle für die Bauchhöhle verbunden zu werden.

6. Instrument nach einem der Ansprüche 1 bis 5, dadurch gekennzeichnet, daß das Aufhängungselement (6) mit der Ebene des Rings (2) einen spitzen Winkel bildet.

7. Instrument nach einem der Ansprüche 2 bis 6, dadurch gekennzeichnet, daß der im wesentlichen in einer Ebene angeordnete, ringförmige Abschnitt (2) mit dem Aufhängungselement (6) durch ein krummliniges Segment (8) verbunden ist, welches aus der Ebene des Rings zu der Seite hin hinausragt, welche derjenigen gegenüberliegt, zu der hin das Aufhängungselement hinausragt.

8. Instrument nach einem der Ansprüche 2 bis 6, dadurch gekennzeichnet, daß es einen Griff oder dgl. aufweist, welcher eine einfache Handhabung des Instruments während der Einführungsphase des Rings in das Innere der Bauchhöhle ermöglicht.

9. Instrument nach einem der Ansprüche 2 bis 8, dadurch gekennzeichnet, daß es aus zwei Abschnitten gebildet ist, welche in einer in Höhe des Aufhängungselements lösbaren Weise miteinander verbindbar sind.

10. Instrument nach Anspruch 1, dadurch gekennzeichnet, daß es einen zum Durchdringen der Bauchwand bestimmten, rohrförmigen, geraden Schenkel (22) umfaßt, der an einem seiner Enden von einem mit ihm einen Winkel bildenden, zum Anliegen an der Innenseite der Bauchwand bestimmten Schenkel (23) verlängert ist und an seinem anderen Ende von einem in einem Eingriffsmittel endenden Aufhängungsarm (24) verlängert ist, wobei der rohrförmige Schenkel (22) als Aufnahme und als Drehungs-Führung für eine Welle (25) dient, deren eines Ende wenigstens einen Schenkel (26) aufweist, welcher beim Schwenken der Welle (25) zwischen einer Stellung, in der er den Schenkel (23) berührt, und einer Stellung bewegbar ist, in der er einen Winkel mit diesem bildet, und deren anderes Ende einen aus dem rohrförmigen Schenkel (22) hinausragenden Betätigungshebel (27) aufweist.
